# EUROPEAN PATENT SPECIFICATION

(11) **EP 1 902 746 B1**
(45) Date of publication and mention of the grant of the patent: **24.06.2009**
(21) Application number: 07253686.5
(22) Date of filing: 18.09.2007
(51) Int. Cl.: A61M 25/01

(54) **Catheter actuator**
Katheter-Aktuator
Actionneur de cathéter

(30) Priority: 21.09.2006 US 846561 P
(43) Date of publication of application: 26.03.2008
(73) Proprietor: Cathrx Ltd, Homebush Bay, NSW 2127 (AU)
(72) Inventor: Ogle, David, Cowan, New South Wales 2081 (AU)
(74) Representative: Nettleton, John Victor

(56) References cited:
- EP-A- 1 302 178
- WO-A-97/42867
- US-A1- 2003 109 778
- US-A1- 2004 116 864

## Description

This invention relates, generally, to a catheter and, more particularly, to a catheter actuator, to a catheter handle assembly including the actuator and to a catheter assembly.

### Background

In the field of heat treatment of tissue, it is desirable if the device heating the tissue is in contact only with the tissue being treated and not surrounding tissue or bodily fluids. This minimises the power required to heat the tissue and also minimises unnecessary damage to other tissue, structures or fluid.

In addition, it is often necessary to overcome tissue irregularities at a site in a patient's body being heat treated. An example where a site in a patient's body is subjected to heat treatment is in the treatment of heart arrhythmias where tissue is ablated in an effort to cure the arrhythmia. The tissue is ablated to create a lesion to block the electrical impulses causing the arrhythmia. To ensure that a lesion of adequate depth is formed, it is desirable that the ablating electrode make good contact with the tissue. Other examples of the use of heat treatment at a site in a patient's body include treatment of Parkinson's disease, tumour ablation, endometriosis and pain management.

Further, a lesion formed by a burn between two spaced electrodes can be more effective than a lesion created between a single electrode and a back plate. The reason for this is that a longer, shallower lesion has a greater likelihood of ablating the affected tissue than a shorter, deeper lesion.

It would be desirable to provide a simple to use actuator to enable a clinician to deploy the electrodes at a distal end of a catheter to enable wide area ablation to be effected.

European Patent Application No. 1 302 178 discloses a system for delivering at least one medical device to a desired location for treatment, and then selectively, deploying the medical device in position, includes an improved handle. The handle is able to selectively hold the delivery system components at any desired configuration during deployment and positioning of the medical device. The handle is also able to operate in more than one mode in which the deployment of the medical device can occur at more than one speed.

### Summary

According to a first aspect of the invention, there is provided a catheter actuator which includes
at least two carriers displaceably arranged relative to each other, a first catheter sheath component of a catheter sheath assembly being connectable to a first carrier and a second catheter sheath component of the catheter sheath assembly being connectable to a second carrier; and
a control mechanism carried, in use, by a catheter handle and associated with the carriers to effect relative displacement between the carriers to cause displacement of a distal end of at least the first catheter sheath component between a non-deployed position and a deployed position characterised in that the second carrier is received substantially in the first carrier and in that the control mechanism includes a movement control member mounted fast with the catheter handle, the control mechanism being operable to control displacement of a combination of the first carrier and the second carrier relative to a further component of the catheter, at least a part of which is fast with a body of the catheter handle.

The distal end of the first catheter sheath component, which may be an electrode sheath of a catheter sheath assembly, may define a plurality of petals or leaves which, in their non-deployed position or configuration, extend parallel to a longitudinal axis of the electrode sheath. In the deployed position or configuration of the distal end of the electrode sheath, the leaves or petals may project radially outwardly relative to the longitudinal axis of the electrode sheath to expose at least one electrode carried on operatively inner surface of the leaves of the electrode sheath.

The second catheter sheath component may be a sleeve of the catheter sheath assembly which is co-axially arranged about the electrode sheath. By moving the sleeve in a proximal direction relative to the electrode sheath an outward force is imparted to the leaves of the electrode sheath to cause them to move to their deployed position and vice versa. The sleeve may have leaves or petals arranged outwardly of, and in register with, those of the electrode sheath.

The further component may be a stylet of the catheter assembly. More particularly, the stylet may be a steering shaft of the catheter assembly.

The first carrier may comprise a body to which the first catheter sheath component is connectable, the body defining a bore. The second carrier may comprise a tubular member slidably received in the bore of the body of the first carrier with the second catheter sheath component being connectable to a distal end of the tubular member.

The second carrier may include a follower which cooperates with the control mechanism for controlling relative displacement between the first carrier and the second carrier, the follower projecting through an elongate opening defined in the body of the first carrier.

The movement control member may be mounted, in use, fast with the catheter handle, the movement control member having a guide arrangement for guiding relative displacement between the first carrier and the second carrier. Further, the control mechanism may include an actuator arm carried by the first carrier and which engages the movement control member, the actuator arm defining a receiving formation in which the follower of the second carrier is received. The actuator arm may be pivotally mounted on the first carrier to be pivotable about a pivot axis.

The actuator arm may carry a cooperating formation which cooperates with the guide arrangement of the movement control member, the cooperating formation being arranged on one side of the pivot axis with the receiving formation being arranged on an opposed side of the pivot axis. The guide arrangement may be a shaped, more particularly a cranked, guide slot defined in the movement control member, the cooperating formation of the actuator arm being a second follower received in the guide slot.

According to a second aspect of the invention, there is provided a catheter handle assembly which includes
a handle body; and
a catheter actuator, as described above, carried by the handle body.

The handle body may define a mounting arrangement for mounting the at least part of the further component of the catheter. A guide tube arrangement may be carried within the handle body for guiding the further component.

The assembly may include a catheter sheath projection arrangement carried on a distal end of the handle body. The handle body may be elongate and in which the catheter sheath projection arrangement is axially displaceably arranged on the distal end of the handle body.

The catheter actuator may be arranged within the catheter sheath projection arrangement. The movement control member of the catheter actuator may be arranged in a fixed position relative to the catheter sheath projection arrangement in a bore at the distal end of the handle body. The movement control member may be mounted on a distal part of a steering control mechanism of the catheter handle assembly and the catheter sheath projection arrangement may be axially displaceably arranged on the distal end of the handle body. Further, the body of the first carrier of the catheter actuator may be fast with the catheter sheath projection arrangement.

The invention extends also to a catheter assembly which includes
a catheter handle assembly as described above;
a stylet received in the handle body of the catheter handle assembly to project through a distal end of the handle body; and
a catheter sheath assembly mounted on, and extending distally from, the catheter handle assembly.

The stylet may be a steering shaft received in a lumen of the catheter sheath assembly. The steering shaft may have a first member fixed within the handle body and a second member axially displaceably arranged relative to the first member.

The first member and the second member may be secured together at a distal region of the steering shaft and one of the first and second members may define a bend enhancing region so that, when there is axial displacement of the first member and the second member relative to each other, bending at the distal region of the steering shaft results.

An electrode may be carried on a distal end of the steering shaft.

The catheter actuator may be configured to displace the first catheter sheath component and the second catheter sheath component by a first amount relative to one another and the catheter sheath assembly, comprising the combination of the first catheter sheath component and the second catheter sheath component, by a second, greater amount relative to the steering shaft so that, when the first catheter sheath component is in its deployed position, the electrode of the steering shaft is operatively positioned relative to the deployed first catheter sheath component.

As described above, when the first catheter sheath component, i.e. the electrode sheath, is in its deployed position, its petals extend substantially radially outwardly. Thus, "operatively positioned" means that the electrode of the steering shaft lies substantially in the same plane as the electrodes of the leaves of the electrode sheath.

When the distal end of the at least first catheter sheath component is in its non-deployed configuration, the distal end of the at least first catheter sheath component may lie proximally of the electrode of the steering shaft.

### Brief Description of Drawings

Embodiments of the present invention are now described by way of example with reference to the accompanying drawings in which:-
Fig. 1 shows a schematic, side view of a catheter actuator, in accordance with an embodiment of the invention, the actuator being in a first position;
Fig. 2 shows a schematic, side view of the actuator in a second position;
Fig. 3 shows a three dimensional view of the actuator in its first position;
Fig. 4 shows a three dimensional view of the actuator in its second position;
Fig. 5 shows a schematic, sectional side view of a distal part of a catheter handle assembly, in accordance with a further embodiment of the invention, with a actuator of the assembly in a first position;
Fig. 6 shows a schematic, sectional side view of the distal part of the catheter handle assembly with the actuator in a second position;
Fig. 7 shows a three dimensional view of a catheter handle assembly;
Fig. 8 shows a schematic, sectional side view of a distal part of a catheter sheath assembly of a catheter assembly in accordance with a further embodiment of the invention with the catheter sheath assembly being in a first, non-deployed configuration; and
Fig. 9 shows a schematic, sectional side view of the distal part of the catheter sheath assembly in a second, deployed configuration.

### Detailed Description of Exemplary Embodiments

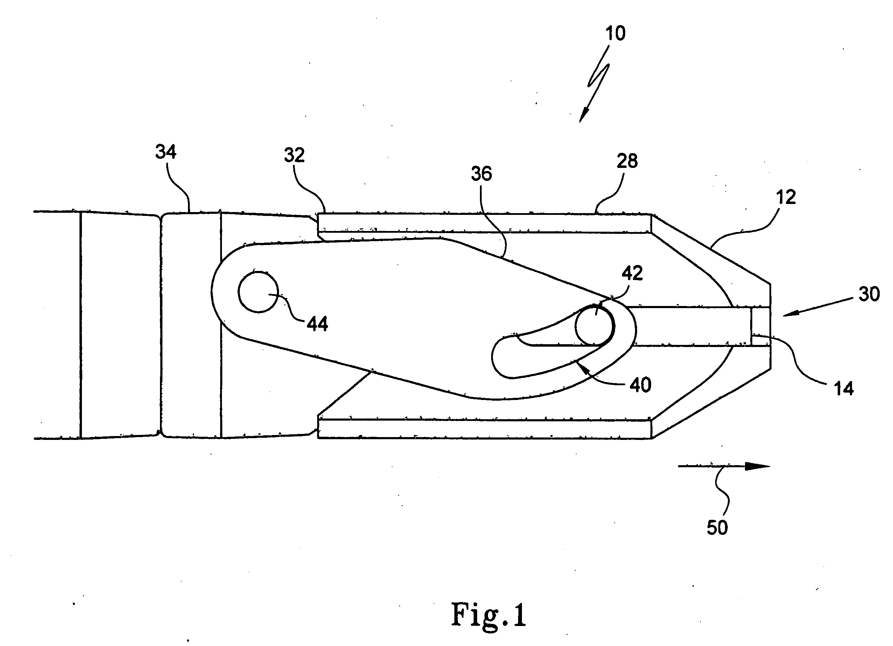

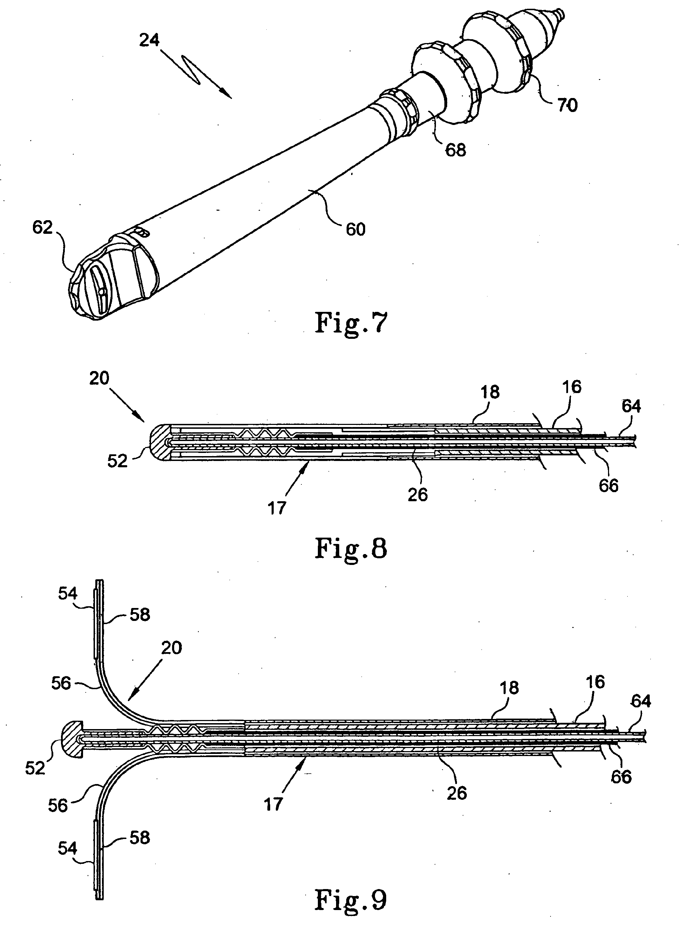

Referring initially to Figs. 1 to 4 of the drawings, a catheter actuator is illustrated and is designated generally by the reference numeral 10. The actuator 10 includes a first carrier 12 and a second carrier 14 displaceably arranged relative to each other. A first catheter sheath component in the form of an electrode sheath 16 of a catheter sheath assembly 17 (Figs. 8 and 9) is securable to a distal end of the first carrier 12 and a second catheter sheath component, in the form of a sleeve 18 (Figs. 8 and 9) of the catheter sheath assembly 17 is securable to a distal end of the second carrier 14.

The catheter sheath assembly 17 has a distal end 20 which is displaceable between a non-deployed configuration as shown in Fig. 8 of the drawings and a deployed configuration as shown in Fig. 9 of the drawings. The movement of the distal end 20 of the catheter sheath assembly 17 between its deployed and non-deployed configurations is controlled by relative movement of the carriers 12 and 14, as will be described in greater detail below.

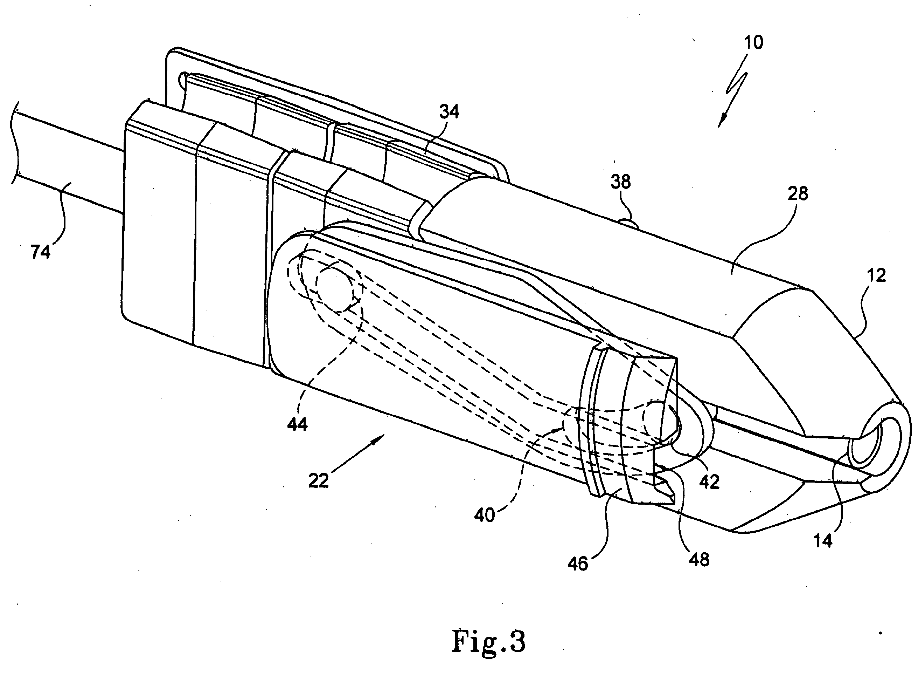

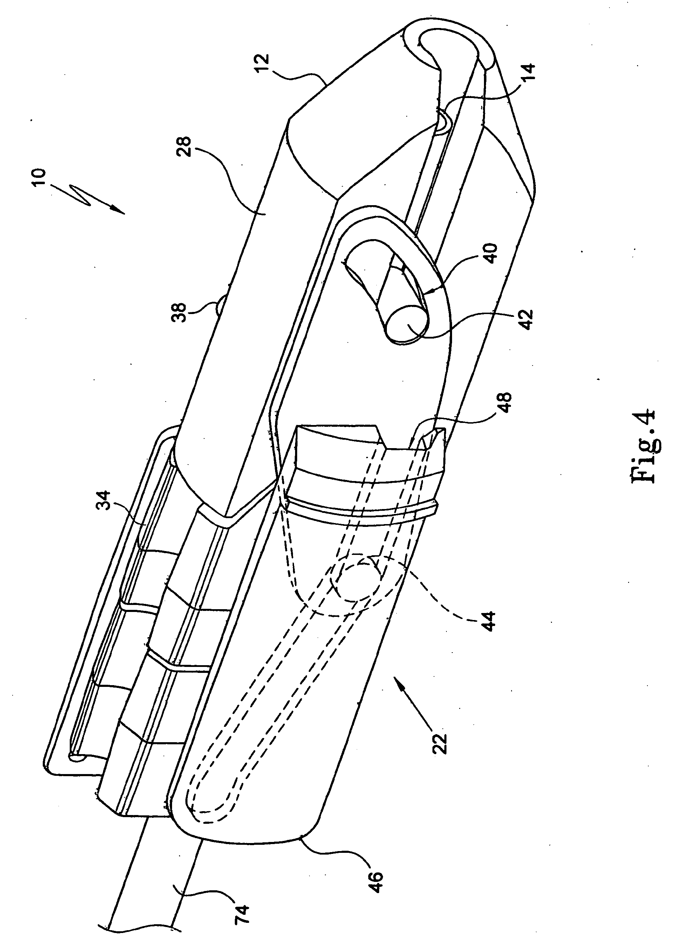

The actuator 10 includes a control mechanism 22 (Figs. 3 & 4) carried, in use, by a catheter handle 24 (Fig. 7). The control mechanism 22 effects relative displacement between the carriers 12 and 14 to cause displacement of the distal end 20 of the catheter sheath assembly 17 between the non-deployed configuration and the deployed configuration.

Both the first carrier 12 and the second carrier 14 are, in turn, arranged in the catheter handle 24 so as to be displaceable relative to a further component, being a steering shaft 26 (Figs. 8 and 9) of a catheter assembly.

The carrier 12 is in the form of a body 28 defining a bore 30. A proximal end 32 of the body 28 is shaped to accommodate a boss 34, the body 28 being fast with the boss 34. The boss 34 is used to support a part of the electrode sheath 16 functioning as a cable for connection to a source of RF energy. This part of the electrode sheath 16 is omitted from the drawings for the sake of clarity. The boss 34 also supports an irrigation conduit (not shown) of the catheter assembly, where applicable. The electrode sheath 16 is secured to the boss 34.

The sleeve 18, in turn, is secured to a distal end of the second carrier 14. It will be appreciated that the electrode sheath 16 passes through the second carrier 14 to be secured to the boss 34.

The actuator 10 includes a control mechanism 22 (Figs. 3 and 4). The control mechanism 22 comprises an actuator arm 36 pivotally mounted on the body 28 of the first carrier 12. The actuator arm 36 is pivotally mounted to the body 28 via a pivot pin 38 (Figs. 3 and 4) to define a pivot axis about which the actuator arm 36 pivots, in use.

An arcuate slot 40 is defined in a distal portion of the actuator arm 36, distally of the pivot pin 38. A follower 42, which is fast with, and projects radially outwardly from, the tubular member of the second carrier 14, is received in the arcuate slot 40.

A second follower 44 is arranged on the actuator arm 36 proximally of the pivot pin 38. The control mechanism 22 includes a movement control member in the form of a plate 46 (Figs. 3 and 4) which, in use, is fast with a distal part of the handle body 24. The plate 46 defines a shaped slot 48. More particularly, the slot 48 is cranked and the follower 44 is received in the slot 48. The shape of the slot 48 dictates the manner in which the catheter sheath assembly 17 moves from its non-deployed configuration to its deployed configuration and vice versa. Further, it will be appreciated that, instead of the plate and slot, the movement control member could adopt other configurations which control the deployment of the distal end 20 of the catheter sheath assembly 17. For example, the movement control member could be a fixed cam having a predefined cam surface with at least the follower 44 of the actuator arm 36 being biased to follow the cam surface of the cam.

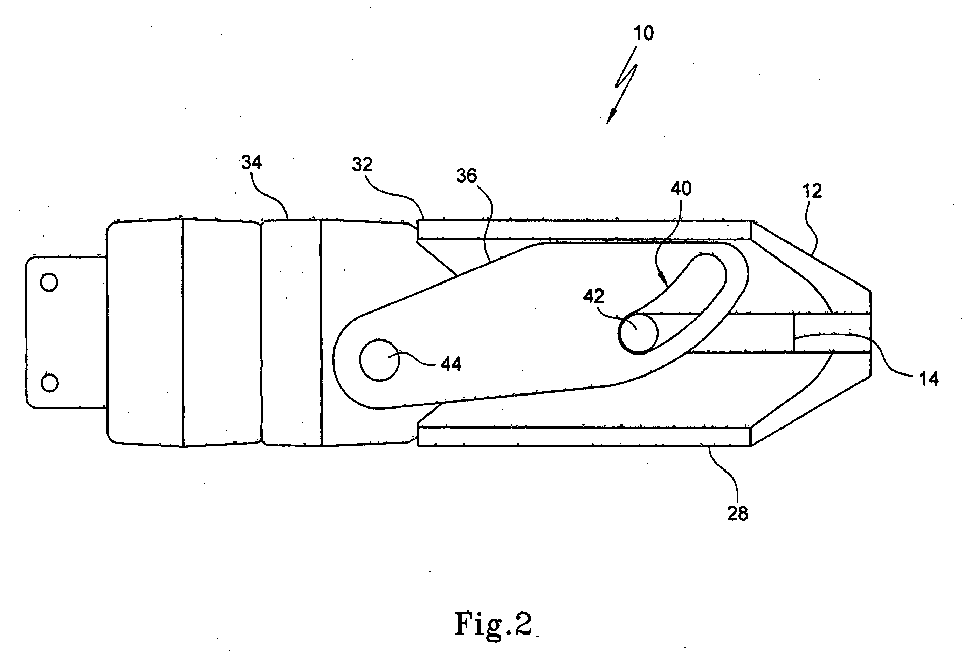

The arrangement is such that, when the first carrier 12 is displaced in a direction of arrow 50 (Fig. 1), the first carrier 12 moves by a predetermined amount relative to the second carrier 14. The amount by which the first carrier 12 moves relative to the second carrier 14 is shown by the relative positions of the carriers 12 and 14 in Figs. 1 and 2 of the drawings and, similarly, in Figs. 3 and 4 of the drawings.

The first carrier 12 and the second carrier 14 both move in the direction of the arrow 50 relative to the steering shaft 26 (Figs. 8 and 9).

Referring again to Figs. 8 and 9, it is to be noted that an electrode 52 is carried on a distal end of the steering shaft 26. Substantially planar electrodes 54 are carried on operatively inner surfaces of petal portions 56 at a distal end of the electrode sheath 16 of the catheter sheath assembly 17. The sleeve 18 of the catheter sheath assembly 17 has similar leaves 58 arranged in register with the petals 56 of the electrode sheath 16. Relative movement between the electrode sheath 16 and the sleeve 18 causes the petals 56 of the electrode sheath 16 and the leaves 58 of the sleeve 18 to move from the position shown in Fig. 8 of the drawings to the position shown in Fig. 9 of the drawings. More particularly, this occurs when the sleeve 18 is displaced proximally relative to the electrode sheath 16.

However, only approximately 3 mm of relative movement between the electrode sheath 16 and the sleeve 18 is required to effect movement of the petals 56 of the electrode sheath 16 from the non-deployed configuration of Fig. 8 to the deployed configuration of Fig. 9. Each petal 56 is approximately 15 mm in length. In order to bring the electrode 52 of the steering shaft 26 more or less into the same plane as the petals 56 in their deployed configuration, approximately 11 mm of movement is required between the steering shaft 26 and the catheter sheath assembly 17. This is effected by moving the combination of the first carrier 12 and the second carrier 14 in the direction of the arrow 50 relative to the steering shaft 26.

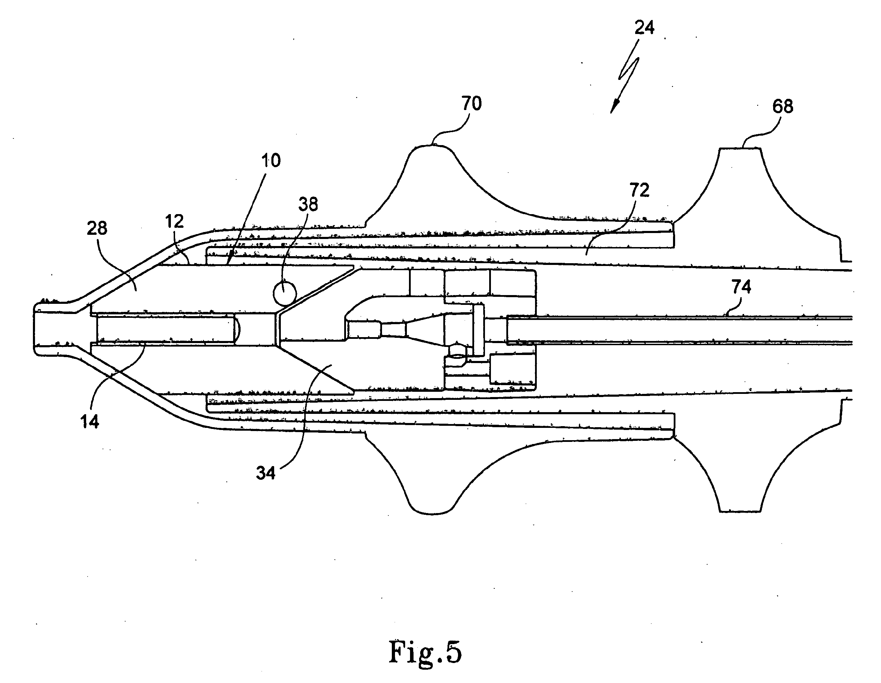

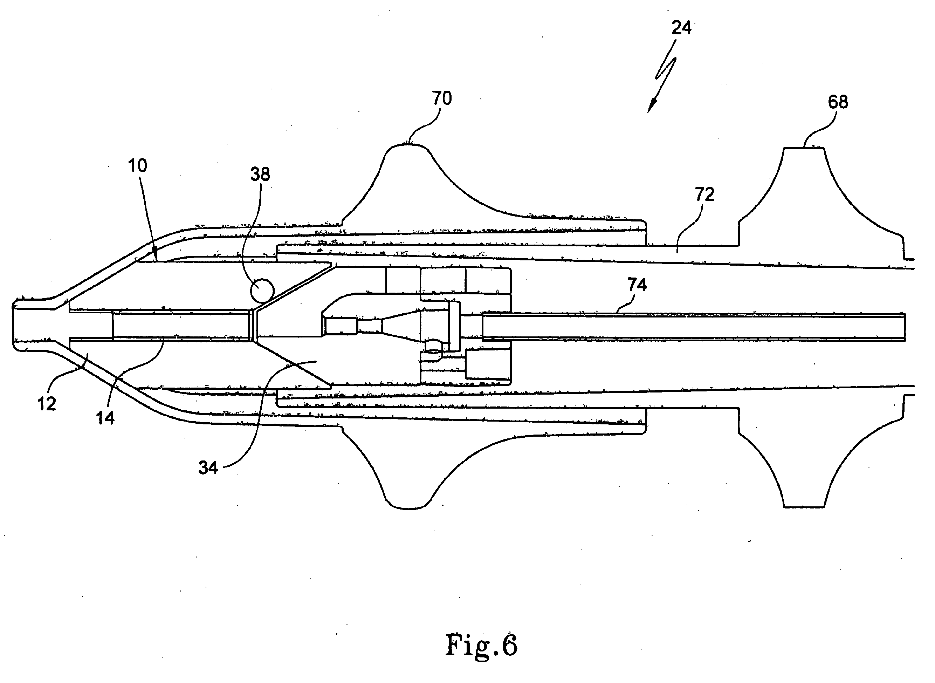

This will be described more clearly below with reference to Figs 5 and 6 which show a distal part of the catheter handle 24. The catheter handle 24 includes a handle body 60 (Fig. 7). A control knob 62 is mounted at a proximal end of the handle body. The control knob 62 mounts a first tubular member 64 (Figs. 8 and 9) of the steering shaft 26. A second tubular member 66 of the steering shaft 26 is co-axially arranged about the first tubular member 64 and is fast with a steering control mechanism 68 carried at a distal end of the handle body 24.

A catheter sheath projection arrangement 70 is carried, in turn, on a distal part of the steering control mechanism 68. Thus, as shown in greater detail in Figs. 5 and 6 of the drawings, a distal part of the steering control mechanism 68 defines a tubular, axially extending part 72 on which the catheter sheath projection arrangement 70 is carried. The body 28 of the first carrier 12 is fast with the catheter sheath arrangement 70. The handle body 24 carries a guide tube arrangement 74 for guiding the steering shaft 26 through the handle body 24.

In order to deploy the petals 56 of the electrode sheath 16, the catheter sheath projection arrangement 70 is moved from the position shown in Fig. 5 of the drawings to the position shown in Fig. 6 of the drawings. When this occurs, the control mechanism 22 of the actuator 10 causes relative displacement between the carriers 12 and 14 to a lesser extent than the displacement of the catheter sheath projection arrangement 70 relative to the steering control mechanism 68. Thus, while the catheter sheath projection arrangement 70 may move approximately 15 mm relative to the steering control mechanism 68 and, consequently, the first carrier 12 moves by the same amount relative to the steering shaft 26, the carrier 14 moves proximally relative to the carrier 12 by a substantially smaller amount, i.e. by about 3 to 4 mm to cause the distal end 20 of the catheter sheath assembly 17 to move from its non-deployed position shown in Fig. 8 of the drawings to its deployed position shown in Fig. 9 of the drawings.

Once the ablation procedure has been completed, the procedure is reversed and the catheter sheath projection arrangement 70 is moved from the position shown in Fig. 6 of the drawings to the position shown in Fig. 5 of the drawings. Once again, relative movement of about 15 mm between the steering control mechanism 68 and the catheter sheath projection arrangement 70 translates into relative movement between the carriers 12 and 14 of some 3 to 4 mm to move the petals 56 of the catheter sheath assembly 17 back to the non-deployed configuration as shown in Fig. 8 of the drawings.

It is a particular advantage of the invention that a catheter actuator is provided which facilitates deployment of a distal end of the catheter sheath assembly in a simple manner using movements with which a clinician would be familiar. The movement of the catheter sheath projection arrangement 70 is an operation with which the clinician would be familiar arising from using a catheter handle as described in the applicant's co-pending International Patent Application No. PCT/AU2006/000266 dated 1 March 2006, entitled "A catheter handle and a catheter assembly including such a handle". A catheter handle of the type described in that patent application provides a benefit in that the clinician has the option of displacing the distal end of an electrode sheath relative to the steering shaft in circumstances where such an action is desirable, eg to obtain access to a difficult to reach site in the patient's body. Thus, by means of a similar movement, the clinician is able to deploy the petals 56 of the catheter sheath 16. Hence, it is a movement which would come naturally to the clinician and facilitates ease of use of the catheter assembly.

Additionally, it is an advantage of the invention that a catheter actuator is provided which enables the catheter sheath assembly to be manipulated between its deployed and non-deployed positions in a one-handed operation.

In addition, the arrangement of the actuator 10 is such that the size of the handle 24 is not increased to any significant extent.

## Claims

1. A catheter actuator (10) which includes
at least two carriers (12, 14) displaceably arranged relative to each other, a first catheter sheath component (16) of a catheter sheath assembly (17) being connectable to the first carrier (12) and a second catheter sheath component (18) of the catheter sheath assembly (17) being connectable to the second carrier (14); and
a control mechanism (22) carried, in use, by a catheter handle (24) and associated with the carriers (12, 14) to effect relative displacement between the carriers (12, 14) to cause displacement, of a distal end of at least the first catheter sheath component (16) between a non-deployed position and a deployed position, **characterised in that** the second carrier (14) is received substantially in the first carrier (12) and **in that** the control mechanism (22) includes a movement control member (46) mounted fast with the catheter handle, the control mechanism being operable to control displacement of a combination of the first carrier (12) and the second carrier (14) relative to a further component (26) of the catheter, at least a part of which is fast with a body of the catheter handle (24).

2. The actuator (10) of claim 1 in which the first carrier (14) comprises a body (28) to which the first catheter sheath component (16) is connectable, the body (28) defining a bore (30).

3. The actuator (10) of claim 2 in which the second carrier (14) comprises a tubular member slidably received in the bore (30) of the body (28) of the first carrier (12) with the second catheter sheath component (18) being connectable to a distal end of the tubular member.

4. The actuator (10) of claim 3 in which the second carrier (14) includes a follower (42) which cooperates with the control mechanism (22) for controlling relative displacement between the first carrier (12) and the second carrier (14).

5. The actuator (10) of claim 4 in which the movement control member (46) is mounted, in use, fast with the catheter handle (24), the movement control member (46) having a guide arrangement (48) for guiding relative displacement between the first carrier (12) and the second carrier (14).

6. The actuator (10) of claim 5 in which the control mechanism (22) includes an actuator arm (36) carried by the first carrier (12) and which engages the movement control member (46), the actuator arm (36) defining a receiving formation (40) in which the follower (42) of the second carrier (14) is received.

7. The actuator (10) of claim 6 in which the actuator arm (36) is pivotally mounted on the first carrier (12) to be pivotable about a pivot axis.

8. The actuator (10) of claim 7 in which the actuator arm (36) carries a cooperating formation (44) which cooperates with the guide arrangement (48) of the movement control member (46), the cooperating formation (44) being arranged on one side of the pivot axis with the receiving formation (40) being arranged on an opposed side of the pivot axis.

9. The actuator (10) of claim 8 in which the guide arrangement (48) is a shaped guide slot defined in the movement control member (46), the cooperating formation (44) of the actuator arm (36) being a second follower received in the guide slot.

10. A catheter handle assembly which includes
a handle body (24); and
a catheter actuator (10), as claimed in claim 9, carried by the handle body (24).

11. The assembly of claim 10 in which the handle body (24) defines a mounting arrangement (62) for mounting the at least part (64) of the further component (26) of the catheter, a guide tube arrangement (74) being carried within the handle body (24) for guiding the further component (26).

12. The assembly of claim 10 or claim 11 which includes a catheter sheath projection arrangement (70) carried on a distal end of the handle body (24), the handle body (24) being elongate and the catheter sheath projection arrangement (70) being axially displaceably arranged on the distal end of the handle body (24).

13. The assembly of claim 12 in which the movement control member (46) of the catheter actuator (10) is arranged in a fixed position relative to the catheter sheath projection arrangement (70) in a bore at the distal end of the handle body (24).

14. The assembly of claim 13 in which the body (28) of the first carrier (12) of the catheter actuator (10) is fast with the catheter sheath projection arrangement (70).

15. A catheter assembly which includes
a catheter handle assembly as claimed in any one of claims 10 to 14;
a stylet (26) received in the handle body (24) of the catheter handle assembly to project through a distal end of the handle body (24); and
a catheter sheath assembly (17) mounted on, and extending distally from, the catheter handle assembly.

16. The catheter assembly of claim 15 in which the stylet (26) is a steering shaft received in a lumen of the catheter sheath assembly (17), the steering shaft 926) having a first member (64) fixed within the handle body and a second member (66) axially displaceably arranged relative to the first member (64).

17. The catheter assembly of claim 16 in which an electrode (52) is carried on a distal end of the steering shaft (26).

18. The catheter assembly of claim 17 in which the catheter actuator (10) is configured to displace the first catheter sheath component (16) and the second catheter sheath component (18) by a first amount relative to one another and the catheter sheath assembly (17), comprising the combination of the first catheter sheath component (16) and the second catheter sheath component (18), by a second, greater amount relative to the steering shaft (26) so that, when the first catheter sheath component (16) is in its deployed position, the electrode (52) of the steering shaft (26) is operatively positioned relative to the deployed first catheter sheath component (16).

19. The catheter assembly of claim 18 in which, when the distal end of the at least first catheter sheath component (16) is in its non-deployed configuration, the distal end of the at least first catheter sheath component (16) lies proximally of the electrode (52) of the steering shaft (26).

## Patentansprüche

1. Katheter-Aktuator (10), der umfasst:
mindestens zwei Träger (12, 14), die verschiebbar relativ zueinander angeordnet sind, wobei ein erstes Katheterhüllenteil (16) einer Katheterhüllenbaugruppe (17) mit dem ersten Träger (12) verbunden werden kann, und wobei ein zweites Katheterhüllenteil (18) der Katheterhüllenbaugruppe (17) mit dem zweiten Träger (14) verbunden werden kann; und
einen Steuerungsmechanismus (22), der bei Benutzung von einem Kathetergriff (24) getragen wird und mit den Trägern (12, 14) in Verbindung steht, um eine relative Verschiebung zwischen den Trägern (12, 14) zu bewirken, um eine Verschiebung eines distalen Endes von mindestens dem ersten Katheterhüllenteil (16) zwischen einer nichtaufgerichteten Position und einer aufgerichteten Position zu veranlassen, **dadurch gekennzeichnet, dass** der zweite Träger (14) im Wesentlichen im ersten Träger (12) aufgenommen wird, und dadurch, dass der Steuerungsmechanismus (22) ein Bewegungssteuerungselement (46) umfasst, das fest mit dem Kathetergriff montiert ist, wobei der Steuerungsmechanismus funktionsfähig ist, um die Verschiebung einer Kombination des ersten Trägers (12) und des zweiten Trägers (14) relativ zu einem weiteren Teil (26) des Katheters zu steuern, von dem mindestens ein Abschnitt am Körper des Kathetergriffes (24) fest ist.

2. Aktuator (10) nach Anspruch 1, bei dem der erste Träger (12) einen Körper (28) aufweist, mit dem das erste Katheterhüllenteil (16) verbunden werden kann, wobei der Körper (28) eine Bohrung (30) definiert.

3. Aktuator (10) nach Anspruch 2, bei dem der zweite Träger (14) ein rohrförmiges Element aufweist, das in der Bohrung (30) des Körpers (28) des ersten Trägers (12) verschiebbar aufgenommen wird, wobei das zweite Katheterhüllenteil (18) mit einem distalen Ende des rohrförmigen Elementes verbunden werden kann.

4. Aktuator (10) nach Anspruch 3, bei dem der zweite Träger (14) ein Eingriffsglied (42) umfasst, das mit dem Steuerungsmechanismus (22) für das Steuern der relativen Verschiebung zwischen dem ersten Träger (12) und dem zweiten Träger (14) zusammenwirkt.

5. Aktuator (10) nach Anspruch 4, bei dem das Bewegungssteuerungselement (46) bei Benutzung fest mit dem Kathetergriff (24) montiert ist, wobei das Bewegungssteuerungselement (46) eine Führungsanordnung (48) für das Führen der relativen Verschiebung zwischen dem ersten Träger (12) und dem zweiten Träger (14) aufweist.

6. Aktuator (10) nach Anspruch 5, bei dem der Steuerungsmechanismus (22) einen Aktuatorarm (36) umfasst, der vom ersten Träger (12) getragen wird, und der mit dem Bewegungssteuerungselement (46) in Eingriff kommt, wobei der Aktuatorarm (36) eine Aufnahmeausbildung (40) definiert, in der das Eingriffsglied (42) des zweiten Trägers (14) aufgenommen wird.

7. Aktuator (10) nach Anspruch 6, bei dem der Aktuatorarm (36) drehbar am ersten Träger (12) montiert ist, damit er sich um eine Drehachse drehen kann.

8. Aktuator (10) nach Anspruch 7, bei dem der Aktuatorarm (36) eine zusammenwirkende Ausbildung (44) trägt, die mit der Führungsanordnung (48) des Bewegungssteuerungselementes (46) zusammenwirkt, wobei die zusammenwirkende Ausbildung (44) auf einer Seite der Drehachse angeordnet ist, wobei die Aufnahmeausbildung (40) auf einer gegenüberliegenden Seite der Drehachse angeordnet ist.

9. Aktuator (10) nach Anspruch 8, bei dem die Führungsanordnung (48) ein geformter Führungsschlitz ist, der im Bewegungssteuerungselement (46) definiert wird, wobei die zusammenwirkende Ausbildung (44) des Aktuatorarmes (36) ein zweites Eingriffsglied ist, das im Führungsschlitz aufgenommen wird.

10. Kathetergriffbaugruppe, die umfasst:
einen Griffkörper (24); und
einen Katheter-Aktuator (10) nach Anspruch 9, der vom Griffkörper (24) getragen wird.

11. Baugruppe nach Anspruch 10, bei der der Griffkörper (24) eine Montageanordnung (62) für das Montieren mindestens des Abschnittes (64) des weiteren Teils (26) des Katheters definiert, wobei eine Führungsrohranordnung (74) innerhalb des Griffkörpers (24) für das Führen des weiteren Teils (26) vorhanden ist.

12. Baugruppe nach Anspruch 10 oder Anspruch 11, die eine Katheterhüllenvorsprungsanordnung (70) umfasst, die an einem distalen Ende des Griffkörpers (24) getragen wird, wobei der Griffkörper (24) länglich ist, und wobei die Katheterhüllenvorsprungsanordnung (70) axial verschiebbar am distalen Ende des Griffkörpers (24) angeordnet ist.

13. Baugruppe nach Anspruch 12, bei der das Bewegungssteuerungselement (46) des Katheter-Aktuators (10) in einer unveränderlichen Position relativ zur Katheterhüllenvorspungsanordnung (70) in einer Bohrung am distalen Ende des Griffkörpers (24) angeordnet ist.

14. Baugruppe nach Anspruch 13, bei der der Körper (28) des ersten Trägers (12) des Katheter-Aktuators (10) mit der Katheterhüllenvorsprungsanordnung (70) fest ist.

15. Katheterbaugruppe, die umfasst:
eine Kathetergriffbaugruppe nach einem der Ansprüche 10 bis 14;
ein Stilett (26), das im Griffkörper (24) der Kathetergriffbaugruppe aufgenommen wird, um durch ein distales Ende des Griffkörpers (24) herauszuragen; und
eine Katheterhüllenbaugruppe (17), die an der Kathetergriffbaugruppe montiert ist und sich distal davon erstreckt.

16. Katheterbaugruppe nach Anspruch 15, bei der das Stilett (26) eine Lenkwelle ist, die in einem Hohlraum der Katheterhüllenbaugruppe (17) aufgenommen wird, wobei die Lenkwelle (26) ein erstes Element (64), das innerhalb des Griffkörpers befestigt ist, und ein zweites Element (66) aufweist, das axial verschiebbar relativ zum ersten Element (64) angeordnet ist.

17. Katheterbaugruppe nach Anspruch 16, bei der eine Elektrode (52) an einem distalen Ende der Lenkwelle (26) getragen wird.

18. Katheterbaugruppe nach Anspruch 17, bei der der Katheter-Aktuator (10) ausgebildet ist, um das erste Katheterhüllenteil (16) und das zweite Katheterhüllenteil (18) um ein erstes Maß relativ zueinander zu verschieben, und die Katheterhüllenbaugruppe (17), die die Kombination des ersten Katheterhüllenteils (16) und des zweiten Katheterhüllenteils (18) aufweist, um ein zweites größeres Maß relativ zur Lenkwelle (26) so, dass, wenn sich das erste Katheterhüllenteil (16) in seiner aufgerichteten Position befindet, die Elektrode (52) der Lenkwelle (26) relativ zum aufgerichteten ersten Katheterhüllenteil (16) operativ positioniert wird.

19. Katheterbaugruppe nach Anspruch 18, bei der, wenn sich das distale Ende des mindestens ersten Katheterhüllenteils (16) in seiner nichtaufgerichteten Konfiguration befindet, das distale Ende des mindestens ersten Katheterhüllenteils (16) in unmittelbarer Nähe der Elektrode (52) der Lenkwelle (26) liegt.

## Revendications

1. Actionneur de cathéter (10), englobant:
au moins deux supports (12, 14) agencés de sorte à pouvoir être déplacés l'un par rapport à l'autre, un premier composant de la gaine du cathéter (16) d'un assemblage de gaine du cathéter (17) pouvant être connecté au premier support (12), et un deuxième composant de la gaine du cathéter (18) de l'assemblage de gaine du cathéter (17) pouvant être connecté au deuxième support (14) ; et
un mécanisme de commande (22), supporté en service par une poignée du cathéter (24) et associé aux supports (12, 14) pour assurer un déplacement relatif entre les supports (12, 14), afin d'entraîner un déplacement d'une extrémité distale, au moins du premier composant de la gaine du cathéter (16), entre une position non déployée et une position déployée, **caractérisé en ce que** le deuxième support (14) est reçu pratiquement dans le premier support (12) et **en ce que** le mécanisme de commande (22) englobe un élément de commande du déplacement (46), monté fermement sur la poignée du cathéter, le mécanisme de commande étant destiné à contrôler le déplacement d'une combinaison du premier support (14) et du deuxième support (14) par rapport à un composant additionnel (26) du cathéter, dont au moins une partie est fixée fermement sur un corps de la poignée du cathéter (24).

2. Actionneur (10) selon la revendication 1, dans lequel le premier support (12) comprend un corps (28) auquel le premier composant de la gaine du cathéter (16) peut être connecté, le corps (28) définissant un alésage (30).

3. Actionneur (10) selon la revendication 2, dans lequel le deuxième support (14) comprend un élément tubulaire, reçu de manière coulissante dans l'alésage (30) du corps (28) du premier support (12), le deuxième composant de la gaine du cathéter (18) pouvant être connecté à une extrémité distale de l'élément tubulaire.

4. Actionneur (10) selon la revendication 3, dans lequel le deuxième support (14) englobe un élément prolongateur (42), coopérant avec le mécanisme de commande (22) pour contrôler le déplacement relatif entre le premier support (12) et le deuxième support (14).

5. Actionneur (10) selon la revendication 4, dans lequel l'élément de commande du déplacement (46) est monté en service fermement sur la poignée du cathéter (24), l'élément de commande du déplacement (46) comportant une structure de guidage (48) pour guider le déplacement relatif entre le premier support (12) et le deuxième support (14).

6. Actionneur (10) selon la revendication 5, dans lequel le mécanisme de commande (22) englobe un bras d'actionnement (36) supporté par le premier support (12) et s'engageant dans l'élément de commande du déplacement (46), le bras d'actionnement (36) définissant une structure de réception (40) dans laquelle est reçu l'élément prolongateur (42) du deuxième support (14).

7. Actionneur (10) selon la revendication 6, dans lequel le bras d'actionnement (36) est monté de manière pivotante sur le premier support (12) en vue d'un pivotement autour d'un axe de pivotement.

8. Actionneur (10) selon la revendication 7, dans lequel le bras d'actionnement (36) supporte une structure de coopération (44), coopérant avec la structure de guidage (48) de l'élément de commande du déplacement (46), la structure de coopération (44) étant agencée sur un côté de l'axe de pivotement, la structure de réception (40) étant agencée sur un côté opposé de l'axe de pivotement.

9. Actionneur (10) selon la revendication 8, dans lequel la structure de guidage (48) est constituée par une fente de guidage profilée définie dans l'élément de commande du déplacement (46), la structure de coopération (44) du bras d'actionnement (36) constituant un deuxième élément prolongateur reçu dans la fente de guidage.

10. Assemblage de poignée de cathéter, englobant :
un corps de poignée (24) ; et
un actionneur du cathéter (10) selon la revendication 9, supporté par le corps de la poignée (24).

11. Assemblage selon la revendication 10, dans lequel le corps de la poignée (24) définit un ensemble de montage (62) pour monter la au moins une partie (64) du composant additionnel (26) du cathéter, un ensemble de tube de guidage (74) étant supporté dans le corps de la poignée (24) pour guider le composant additionnel (26).

12. Assemblage selon les revendications 10 ou 11, englobant une structure faisant saillie de la gaine du cathéter (70), supportée sur une extrémité distale du corps de la poignée (24), le corps de la poignée (24) étant allongé et la structure faisant saillie de la gaine du cathéter (70) étant agencée de sorte à pouvoir être déplacée axialement sur l'extrémité distale du corps de la poignée (24).

13. Assemblage selon la revendication 12, dans lequel l'élément de commande du déplacement (46) de l'actionneur du cathéter (10) est agencé dans une position fixe par rapport à la structure faisant saillie de la gaine du cathéter (70), dans un alésage au niveau de l'extrémité distale du corps de la poignée (24).

14. Assemblage selon la revendication 13, dans lequel le corps (28) du premier support (12) de l'actionneur du cathéter (10) est fixé fermement sur la structure faisant saillie de la gaine du cathéter.

15. Assemblage de cathéter, englobant :
un assemblage de poignée de cathéter selon l'une quelconque des revendications 10 à 14 ;
un stylet (26) reçu dans le corps de la poignée (24) de l'assemblage de la poignée du cathéter, destiné à déborder à travers une extrémité distale du corps de la poignée (24) ; et
un assemblage de gaine du cathéter (17) monté sur l'assemblage de poignée du cathéter et débordant dans une direction distale de celui-ci.

16. Assemblage de cathéter selon la revendication 15, dans lequel le stylet (26) constitue un arbre de direction reçu dans une lumière de l'assemblage de gaine du cathéter (17), l'arbre de direction (26) comportant un premier élément (64) fixé dans le corps de la poignée, et un deuxième élément (66) agencé de sorte à pouvoir être déplacé axialement par rapport au premier élément (64).

17. Assemblage de cathéter selon la revendication 16, dans lequel une électrode (52) est supportée sur une extrémité distale de l'arbre de direction (26).

18. Assemblage de cathéter selon la revendication 17, dans lequel l'actionneur du cathéter (10) est configuré de sorte à entraîner un déplacement relatif entre le premier composant de la gaine du cathéter (16) et le deuxième composant de la gaine du cathéter (18) sur une première distance, et l'assemblage de gaine du cathéter (17), comprenant la combinaison du premier composant de la gaine du cathéter (16) et du deuxième composant de la gaine du cathéter (18), sur une deuxième distance plus grande par rapport à l'arbre de direction (26), de sorte que lorsque le premier composant de la gaine du cathéter (16) se trouve dans sa position déployée, l'électrode (52) de l'arbre de direction (26) est positionnée de manière opérationnelle par rapport au premier composant déployé de la gaine du cathéter (16).

19. Assemblage de cathéter selon la revendication 18, dans lequel, l'extrémité distale du au moins premier composant de la gaine du cathéter (16) se trouvant dans sa position non déployée, l'extrémité distale du au moins premier composant de la gaine du cathéter (16) se situe en un emplacement proximal par rapport à l'électrode (52) de l'arbre de direction (26).
